# EUROPEAN PATENT APPLICATION

(11) **EP 3 669 912 A1**
(43) Date of publication of application: **24.06.2020**
(21) Application number: 18306739.6
(22) Date of filing: 19.12.2018
(51) Int. Cl.: A61M 5/315, G16H 80/00, G16H 40/60, G16H 20/17, A61M 5/142, A61M 5/172, H04B 7/24

(54) **DRUG DELIVERY DEVICE AND DRUG DELIVERY SYSTEM**

(71) Applicant: Sanofi, 75008 Paris (FR)
(72) Inventor: The designation of the inventor has not yet been filed
(74) Representative: Sanofi Aventis Deutschland GmbH

(57) **Abstract**

The present disclosure relates to a drug delivery system and to a drug delivery device, the drug delivery device (20) comprising:
- a receptacle (23) to accommodate a drug container (10), wherein the drug container (10) is provided with a container identification (52), the container identification (52) being at least indicative of a content of the drug container (10),
- a container identification arrangement (50) configured to detect and/or to identify the container identification (52) when the drug container (10) is arranged in the receptacle (23),
- a delivery device interface (24) configured to transmit uplink data (UL) to a communication device (60), wherein the uplink data (UL) contains at least the container identification (52) obtainable from the container identification arrangement (50) and wherein the delivery device interface (24) is configured to receive downlink data (DL) from the communication device (60) in return to the transmission of the uplink data (UL),
- a drive mechanism (40) operably engageable with the drug container (10) and configured to expel or to dispense at least a dose of the drug from the drug container (10) on the basis of the downlink data (DL) received from the delivery device interface (24).

## Description

The present disclosure relates in one aspect to a drug delivery device, such as a drug injection device or drug infusion device. In a further aspect the disclosure relates to a drug delivery system comprising a drug delivery device and a communication device. In a further aspect the present disclosure relates to a method of adjusting at least one expelling or dispensing parameter of a drug delivery device.

### Background

Drug delivery devices, such as injection devices or infusion devices are configured for setting and dispensing a single or multiple doses of a liquid medicament.

Injection or infusion devices, e.g. in form of pen-type injectors or infusion pumps, have to meet a number of user-specific requirements. For instance, with patient's suffering chronic diseases, such as diabetes, the patient may be physically infirm and may also have impaired vision. Suitable injection or infusion devices especially intended for home medication therefore need to be robust in construction and should be easy to use. Furthermore, manipulation and general handling of the device and its components should be intelligible and easy understandable. Moreover, the dose setting as well as dose dispensing procedure must be easy to operate and has to be unambiguous.

Typically, injection or infusion devices comprise a housing having a receptacle to receive a drug container. The drug or medicament container accommodates a liquid medicament or drug for parenteral administration. Such injection or infusion devices further comprise a drive mechanism by way of which a prescribed dose of the medicament can be expelled or withdrawn from the medicament container.

In order to comply with a medicament treatment schedule it must be ensured that a certain type of drug container containing a certain type of a drug or medicament is appropriately used with a certain type of a suitable drug delivery device. Moreover, it should be ensured, that a drug container to be used with a drug delivery device complies with a given treatment schedule of the person or patient. Drugs or medicaments commercially distributed in a drug container may not only differ with regard to their pharmaceutically active compounds but also with regard to the concentration of a pharmaceutically active compound.

Drug delivery devices and drug delivery systems configured for self or home medication should provide a failure-safe, reliable and failure-proof mechanism to prevent misuse or a wrong use of a given combination of a drug delivery device and a drug container filled with a liquid drug or medicament. The drug delivery device and the drug delivery system should provide an automated adaptation to a given treatment schedule. The drug delivery device and the drug delivery system should provide a high level of user acceptance and ease of use. Moreover, it is desirable to provide an automated and/or dynamic adaptation of drug delivery device settings to modifications of a given treatment schedule.

### Summary

In one aspect there is provided a drug delivery device. The drug delivery device comprises a receptacle. The receptacle is configured to accommodate a drug container. Typically, the drug container is at least partially filled with a liquid drug or medicament. The drug container is provided with a container identification. The container identification is at least indicative of a content of the drug container. Typically, the container identification is at least indicative of the drug or medicament contained inside the drug container.

The drug delivery device further comprises a container identification arrangement. The container identification arrangement is operable or configured to detect and/or to identify the container identification when the drug container is arranged in the receptacle. Hence, the container identification arrangement is configured or operable to extract the container identification from the drug container and to provide the container identification for further processing by the drug delivery device itself or by at least one further device connected to the drug delivery device.

The drug delivery device further comprises a delivery device interface. The delivery device interface is configured or operable to transmit uplink data to a communication device. The uplink data contains at least the container identification that is obtainable from the container identification arrangement. Moreover, the delivery device interface is configured to receive downlink data from the communication device in return to the transmission of the uplink data. The drug delivery device also comprises a drive mechanism that is operably engageable with the drug container. The drive mechanism is configured or operable to expel or to dispense at least a dose of the drug or medicament from the drug container on the basis of the downlink data received from the delivery device interface.

The mutual interaction of the container identification arrangement, the delivery device interface and the drive mechanism provides a rather automated or self-acting recognition of a drug container and/or of a drug, a data exchange with a communication device and a rather automated or self-acting adjustment or calibration of a drive mechanism in accordance with or depending on downlink data received from the communication device.

The communication device is typically a device separate from the drug delivery device. With some examples the communication device is operable to derive or to generate downlink data configured for submission to the drug delivery device directly from uplink data received from the drug delivery device.

With other examples the communication device is exclusively operable to provide a communication link to an external data processing device or data processing entity, such as a database via a network.

The drive mechanism is typically electromechanically implemented. In this way, the expelling or dispensing of the drug from the drug container can be controlled or adjusted electronically. An electronically controllable or electronically adjustable drive mechanism can be easily controlled on the basis of downlink data received on the communication device.

The interaction of the container identification arrangement, the delivery device interface and the drive mechanism enable an automated or self-acting adjustment or calibration of the drive mechanism depending on the container identification. A deployment of implementing rules according to which the drive mechanism is controlled, adjusted or calibrated does not have to be provided by the drug delivery device itself. Such a deployment of implementing rules may be provided by the communication device or by a separate external data processing device. In this way, the electronic implementation of the drug delivery device can be simplified compared to a drug delivery device that provides an onboard control for a container-based adjustment or calibration of a drive mechanism.

The presently proposed external adjustment or calibration of the drive mechanism's control provides and enables a dynamic mapping or assignment of a drive mechanism's control, adjustment or calibration in view of an identified or recognized container identification. The externally processed mapping or assignment of a container identification with a control, adjustment or calibration of the drive mechanism also enables an external modification of the control, adjustment or calibration of the drive mechanism during the lifetime or time of use of the drug container.

In this way, the drug delivery device can be dynamically adapted to modifications of a prescribed treatment schedule. If for instance, the treatment schedule is subject to a modification or change during use of the drug container in the drug delivery device, the downlink data to be received by the delivery device interface can be modified accordingly when responding to the transmission of the uplink data. Depending on the modified downlink data, the drive mechanism is controlled, adjusted or calibrated accordingly. In this way, a particular user interaction may be no longer required in order to modify the settings of the drug delivery device for treatment schedule compliance. Of course, the user may be informed by the drug delivery device when modified downlink data has been received and when the drive mechanism should become subject to a modified control, adjustment or calibration. Here, the user may be given the possibility to prompt such a modification of the device settings and/or of the drive mechanism.

The external mapping and/or assignment provided by the transmission of uplink data to the communication device and by the receiving of downlink data from the communication device is of further benefit to reduce manufacturing costs of the drug delivery device. Basically, the drug delivery device may only comprise the container identification arrangement and the delivery device interface thus enabling the drug delivery device to recognize and to identify a drug container and the drug contained therein, to transmit a container identification and to receive control, adjustment or calibration parameters of the drive mechanism in return.

Typically, the drug delivery device comprises a housing accommodating the container identification arrangement, the delivery device interface and the drive mechanism. The receptacle to accommodate the drug container may be configured as a component of the housing of the drug delivery device. The receptacle may be detachable from the housing or may be moveable relative to the housing in order to enable an exchange of a drug container. With other examples the receptacle accommodating the drug container may be entirely located inside the housing of the drug delivery device. The receptacle may be further equipped with a movable or detachable closure by way of which the receptacle can be closed or sealed. The closure further enables replacement of the drug container.

The drug delivery device may be implemented as a drug injection device. The drug delivery device, the receptacle and/or the housing of the drug delivery device may be provided with a piercing or needle assembly. The piercing or needle assembly may be detachably connectable to the receptacle, to the drug container or to the housing of the drug delivery device. The piercing or needle assembly typically comprises a hollow tipped cannula configured to establish a fluid communication with the interior of the drug container. For instance, the piercing or needle assembly comprises a double-tipped injection needle having a proximal tipped end configured to pierce a seal of the drug container and having a distal end configured to penetrate biological tissue of a patient. The drug delivery device may comprise a pen-type injection device. Here, the housing of the drug delivery device may comprise an elongated shape, e.g. an elongated tubular shape.

When implemented as an injection device the drug container may comprise a tubular-shaped barrel with oppositely located proximal and distal ends. The distal end configured for a fluid transferring connection with the piercing or needle assembly may be provided with a pierceable seal. The proximal end or towards the proximal direction the drug container may be sealed by a bung or piston longitudinally displaceable relative to a barrel of the drug container. Typically, the bung or piston is configured to be urged or driven in the distal direction by a plunger or piston rod of the drive mechanism of the drug delivery device.

With other examples the drug delivery device is implemented as a pump. Here, the drive mechanism may comprise a suction pump and the drug container may comprise a flexible bag or pouch filled with the medicament. The drive mechanism may comprise a peristaltic pump or any other pump configuration by way of which a dose or a rather constant flow of medicament can be withdrawn from the drug container by way of suction. Here, an outlet of the container is typically connectable to a tube or to an infusion line. With an opposite end, such a tube or infusion line may be connected or may be connectable or may be connected to a cannula or to a port system.

According to a further example the drug delivery device comprises a delivery device processor that is connected to the delivery device interface. The delivery device processor is operable to control the drive mechanism on the basis of at least one expelling or dispensing parameter. The processor is operable to process downlink data obtained from the delivery device interface and to adjust or to modify the at least one expelling or dispensing parameter of the drive mechanism on the basis of the downlink data.

Alternatively or additionally the processor is operable to extract at least one of an expelling or dispensing parameter of the drive mechanism from the downlink data. The at least one expelling or dispensing parameter may be indicative of at least one of the following parameters: a point of time at which the drug should be dispensed or expelled, the amount of the drug, the amount of the pharmaceutically active compound of the drug, the velocity of the dose expelling or dose dispensing, a flow rate of the drug and so on. The delivery device processor may be operable to process the downlink data and to adjust or to modify the at least one expelling or dispensing parameter on the basis of the downlink data. Here, the downlink data may be only indicative of an adjustment or modification of an already existing expelling or dispensing parameter of the drive mechanism. Alternatively, the at least one expelling or dispensing parameter may be contained in the downlink data. The delivery device processor may be then operable to extract the at least one expelling or dispensing parameter from the downlink data.

In a further example the delivery device processor may be further configured to control or to manage the communication between the container identification arrangement and the delivery device interface. Hence, the delivery device processor may be configured to receive the container identification provided by the container identification arrangement and to forward the container identification arrangement to the delivery device interface. The delivery device processor may be further configured to transfer the container identification into uplink data or to generate uplink data from the container identification. Insofar, the delivery device processor may be operable to generate the uplink data and to trigger the delivery device interface to transmit the uplink data to the communication device.

Moreover and according to another example, the delivery device processor may be further operable to control, to adjust or to calibrate the drive mechanism in accordance to the downlink data received from the delivery device interface. The control, the adjustment or the calibration of the drive mechanism may be governed by the at least one expelling or dispensing parameter. Hence, adjusting, modifying or extracting the at least one expelling or dispensing parameter may automatically lead to a respective control, adjustment or calibration of the drive mechanism, when the drive mechanism is operated on the basis of the at least one expelling or dispensing parameter.

According to a further example the drug delivery device also comprises a delivery device storage configured to store at least one or more of at least a portion of the container identification, at least a portion of the uplink data and at least a portion of the downlink data. Optionally, the delivery device storage may be configured or operable to store the at least one expelling or dispensing parameter.

The delivery device storage provides buffering of at least one of the container identification, at least a portion of the uplink data and at least a portion of the downlink data. In situations, where a communication link to a communication device is not provided or temporarily interrupted, the container identification provided by the container identification arrangement may be stored in the delivery device storage. If a communication link to the communication device should become available, the container identification can be read from the delivery device storage and can be transmitted to the communication device via the uplink data. Depending on whether the container identification has already been implemented into uplink data also the uplink data or at least a portion thereof may be stored in the delivery device storage. This enables processing of container identification and embedding of container identification into the uplink data independent of the availability of a communication link to the communication device.

Storing of at least a portion of the downlink data and/or storing of at least one expelling or dispensing parameter in the delivery device storage is of particular benefit to conduct or to process a control, an adjustment or a calibration of the drive mechanism in situations where a communication link to the communication device is not available or interrupted.

The delivery device storage may be implemented as non-volatile storage. Data stored in the delivery device storage may persist even when the drug delivery device has been switched off. If the drug delivery device is implemented as a portable drug delivery device a switching off enables a reduction of electric power consumption. When the drug delivery device is switched on, an automated control, adjustment or calibration of the drive mechanism may be conducted on the basis of data stored in the delivery device storage, e.g. on the basis of the downlink data and/or on the basis of the at least one expelling or dispensing parameter stored in the delivery device storage. Typically, the delivery device processor is operable to write data into the delivery device storage and to read respective data from the delivery device storage. An automated or self-acting control, adjustment or calibration of the drive mechanism may be triggered by the delivery device processor.

According to another example the drug delivery device further comprises a patient identifying arrangement. The patient identifying arrangement is connected to at least one of the delivery device processor and the delivery device interface. The patient identifying arrangement may be connected to both, the delivery device processor and the delivery device interface. The patient identifying arrangement is operable to identify a patient using the drug delivery device.

The patient identifying arrangement is also operable to generate a patient identifier and to incorporate the patient identifier into the uplink data. For this the patient identifying arrangement is either operable to modify the uplink data, e.g. provided by the delivery device processor and to forward the modified uplink data to the delivery device interface. The modified uplink data contains information about the identity of the patient being identified by the patient identifying arrangement.

With another example the patient identifying arrangement is operable to generate the patient identifier in response to a patient identifying procedure conducted by the patient identifying arrangement. When the patient identifier has been generated by the patient identifying arrangement the patient identifying arrangement may be operable to transmit the patient identifier to at least one of the delivery device processor and the delivery device interface. The respective component or entity receiving the patient identifier, i.e. the delivery device processor and/or the delivery device interface is/are then operable to process the patient identifier and to incorporate the patient identifier into the uplink data.

The patient identifying arrangement may be accessible from outside the drug delivery device. The patient identifying arrangement may be attached to the housing of the drug delivery device. It may also be integrated into the housing or may be located inside the housing but may be accessible from outside the housing. The patient identifying arrangement may be implemented in many different ways. The patient identifying arrangement typically comprises at least one of a display, a touchscreen, a button or a dial, a sensor, e.g. a fingerprint sensor, an optical sensor, a camera, a biometric scanner or the like.

Depending on the specific implementation of the patient identifying arrangement a user may be prompted to enter a user identification, a passphrase or password or a code. The code may be a simple number or character code. The code may also be provided as an optical code, such as a barcode or a two-dimensional coding pattern, such as a data matrix code. For this, the patient identifying arrangement may comprise a scanner operable to scan a visual code.

The patient identifying arrangement may further comprise a code reader operable to read an optical code, a magnetic code or an electromagnetic code. Here, the patient identifying arrangement may comprise a radio-frequency reader, such as an RFID reader or a near field communication (NFC) device operable to establish an NFC communication link between an NFC or RFID readable code provided by the user and being unique for each user of the drug delivery device.

With the help of the patient identifying arrangement the drug delivery device can be personalized to that particular patient actually using the drug delivery device. By incorporating the patient identifier into the uplink data and by transmitting the uplink data to the communication device, the uplink data can be processed for a specific patient assigned to the patient identifier. Consequently, the downlink data received from the communication device in response to the transmission of the patient specific uplink data can be also patient specific. In this way, the at least one expelling or dispensing parameter and/or the control, adjustment or calibration of the drive mechanism can be adapted to a specific patient identified by the patient identifying arrangement. Since the patient identifier is incorporated into the uplink data and since patient specific expelling or dispensing parameters for the drive mechanism of the drug delivery device can be derived from downlink data patient specific information does not have to be stored in the drug delivery device. In this way, the drug delivery device itself may easily comply with data protection regulations.

Furthermore, the patient identifying arrangement and the uplink and downlink data transmission with a communication device enable a patient specific individual setting, controlling, adjusting and calibrating of the drug delivery device and/or of its drive mechanism. In this way one and the same drug delivery device may be frequently used or shared by different persons, e.g. living in the same household and/or sharing the drug delivery device. For example in diabetes therapy, there is a variety of insulin types, e.g. rapid acting insulins and long acting insulins, GLP1-agonists, and mixtures of insulins and GLP1-agonists that may be used by patients on prescription. There may be more than one patient requiring diabetes therapy in a family, so different medications may be present at the same time in the environment of a patient. With the presently proposed drug delivery device, the patients can be inherently protected from using a wrong medication. The presently described automated adaptation of the dosing mechanism to the correct treatment will protect the patient from getting the wrong dose

With a further example the patient identifying arrangement may be coupled to the delivery device processor. The delivery device processor may be operable to check the identity of a person or patient actually using the drug delivery device. For this, the delivery device processor and the patient identifying arrangement may be operable to communicate with each other. Before a user-induced dose setting or dose dispensing action is actually processed and executed by the delivery device processor, the delivery device processor may be operable to request the identity of the person actually operating the drug delivery device.

Insofar, the delivery device processor may be operable to trigger a patient identifying procedure to be conducted and executed by the patient identifying arrangement. If the patient has successfully passed the patient identifying procedure the delivery device processor may be operable to release the drive mechanism and/or to trigger a drug expelling or drug dispensing procedure. In one example, the patient identifying procedure includes generation of a patient identifier and comparing the patient identifier, e.g. with a patient identifier stored in the delivery device storage.

If the actually generated patient identifier matches with the patient identifier previously stored in the delivery device storage this is interpreted as a confirmation, that the drug delivery device and the drive mechanism has been actually set to the individual settings, adjustments and calibrations of that particular patient actually using the drug delivery device. In another situation, wherein the patient identifier actually generated by the patient identifying arrangement does not match with a patient identifier previously stored in the delivery device storage the patient identifying procedure may further include the step of incorporating the patient identifier into the uplink data, to trigger submission of the uplink data to the communication device and to receive downlink data from the communication device. Consequently, and based on the downlink data the at least one expelling or dispensing parameter of the drive mechanism is modified or extracted, respective parameters and patient individual settings can be stored in the delivery device storage and the entire drug delivery device can be individually set and configured for the patient actually using the drug delivery device.

In another example the delivery device processor is further configured to process at least one physiologic parameter or environmental parameter. The delivery device processor is further configured and/or operable to adjust or to modify the at least one expelling or dispensing parameter of the drive mechanism on the basis of at least one of the physiologic parameter or environmental parameter. The physiologic parameter or environmental parameter may be provided in different ways. The physiologic parameter or environmental parameter may be obtained through a user input, e.g. via a user interface of the drug delivery device. Furthermore, the physiologic parameter or environmental parameter may be provided by the communication device. The physiologic parameter or environmental parameter may be incorporated into the downlink data received from the communication device. Furthermore, the physiologic parameter or environmental parameter may be gathered, determined or measured by the drug delivery device itself. Alternatively, the physiologic parameter or environmental parameter is obtained, determined or measured by an external device or entity and is transmitted to the drug delivery device via the delivery device interface or via a separate interface.

The physiologic parameter may be at least one of the following parameters: body temperature, blood pressure, blood glucose level, heart rate, blood oxygen saturation, etc. Examples of environmental parameters include at least one of the following: outside temperature, humidity, atmospheric pressure, solar radiation etc.

At least one of the physiologic parameter or environmental parameter may be directly processed by the delivery device processor. Depending on the physiologic parameter or environmental parameter the delivery device processor is operable to adjust or to modify the at least one expelling or dispensing parameter. Alternatively and if the physiologic parameter or environmental parameter is provided in the drug delivery device, e.g. if the physiologic parameter or environmental parameter is entered into the drug delivery device or is actually measured by the drug delivery device the physiologic parameter and/or the environmental parameter can be also incorporated into the uplink data and can be hence transmitted to the communication device. Adjusting or modifying of the at least one expelling or dispensing parameter on the basis of the physiologic parameter or environmental parameter may then be conducted by the communication device or by some other external data processing device operable to communicate with the communication device.

In a further example the drug delivery device comprises a sensor assembly configured to measure the at least one physiologic parameter or environmental parameter. The sensor assembly is further configured to transmit the at least one physiologic parameter and/or environmental parameter to the delivery device processor. In particular, the sensor assembly may be operable or configured to transmit the at least one physiologic parameter and/or environmental parameter to the delivery device interface of the drug delivery device.

Alternatively or additionally the sensor assembly may be operable to transmit the at least one physiologic parameter or environmental parameter directly to the communication device. It may be operable to set up a further uplink communication link with, e.g. a communication device interface, which in turn is operable to communicate with both, the delivery device interface and with a respective communication interface of the sensor assembly.

The sensor assembly can be integrated into the drug delivery device. It may be arranged inside the housing of the drug delivery device. It may comprise a sensor extending through the housing of the drug delivery device or having access to the surrounding environment. The sensor assembly is configured to measure or to determine at least one of the above given physiologic parameters and/or environmental parameters. In this way the expelling or dispensing of the drug from the drug container can be individually adapted in accordance to the momentary physiologic or environmental situation the patient is confronted with or exposed to.

In a further example the container identification arrangement comprises at least one of a mechanical sensor, an optical sensor, a capacitive sensor, a magnetic sensor and an electromagnetic sensor, e.g. a radio frequency (RF) sensor. The container identification arrangement is operable to generate and to transmit an electronic identification signal being indicative of the container identification when the drug container is arranged in the receptacle of the drug delivery device. A mechanical sensor may comprise a mechanically encoded switch matching with a mechanical coding provided on the drug container. Only a drug container equipped with a suitable mechanical encoding will be operable to activate or to deactivate the mechanical sensor upon insertion of the drug container in the receptacle. The mechanical sensor may comprise an array of numerous mechanical switches, e.g. arranged in a row or in a two-dimensional array. For this, the drug container may comprise a mechanical encoding of a corresponding geometry. Each drug container containing a different drug formulation or different drug concentration typically has a corresponding mechanical encoding. Drug containers comprising different drug formulations or different drug concentrations comprise different mechanical encodings.

When the drug containers provided with a mechanical encoding, the container identification arrangement of the drug delivery device may comprise an electromechanical switch, e.g. in form of a microswitch configured and/or operable to cooperate with the mechanical encoding of the drug container.

The optical sensor of the container identification arrangement may comprise a photodiode combined with a light source. The drug container may comprise a correspondingly arranged optically transmissive or optically reflective pattern or portion. Upon insertion of the drug container into the receptacle light or electromagnetic radiation transmitted from a light source will be either blocked or reflected by the optical encoding provided on the drug container. The photodiode of the optical sensor may thus receive or detect an optical signal depending on the structure and properties of the optical encoding provided on the drug container. Also here, the optical encoding of the drug container as well as the optical sensor may comprise a certain extension in a longitudinal and/or transverse direction so as to enable a one-dimensional and/or two-dimensional visual encoding. The optical sensor may comprise a barcode reader. The optical sensor may further comprise a scanner configured to scan and to identify a two-dimensional optical code, such as a data matrix code.

The capacitive sensor of the container identification arrangement is typically configured to detect an electrostatic field provided on and by a respective capacitive encoding attached to or integrated into the drug container. Likewise, the drug container may be equipped or provided with a magnetic encoding detectable and readable by a correspondingly configured magnetic sensor of the container identification arrangement. When implemented as an electromagnetic sensor the container identification arrangement is particularly operable to transmit and/or to receive electromagnetic radiation, such as radio-frequency signals or electromagnetic signals of any other frequency band. Here, the encoding provided on the drug container may comprise an electronic circuit configured for wireless signal transmission. The electromagnetic encoding provided on the drug container may include one of an RFID tag or NFC tag. The electromagnetic sensor of the container identification arrangement then comprises a correspondingly configured RFID reader or NFC reader.

According to a further example the drug delivery device comprises the drug container which is arranged in the receptacle. The drug container may comprise a cartridge with a cylindrical barrel sealed in distal direction by a pierceable seal or closure and sealed in proximal direction by a bung or stopper that is moveable in longitudinal direction relative to the barrel. The container identification may be provided on an outside surface of the barrel. It may be embedded inside the barrel. It may be embedded in the sidewall of the barrel or may be located on an inside wall of the barrel. Alternatively or additionally the container identification may be provided on a proximal surface of the stopper or bung facing away from the interior volume of the barrel.

The container identification can be also embedded or integrated into the bulk of the bung or stopper. Depending on the specific type of the container identification implementation it is either visible from outside the drug container or it is invisible from outside. When implemented as a mechanical encoding or optical encoding the container identification is typically visible from outside. When implemented as a capacitive, magnetic or electromagnetic encoding the container identification can be invisibly integrated into the drug container.

With further examples the drug container comprises a flexible bag or pouch filled with a drug or medicament. The drug or medicament may be provided in liquid or lyophilized form inside the interior volume of the drug container. When the drug container is readily assembled inside the receptacle and hence inside the drug delivery device the drug delivery device may be prepared and ready for setting and/or dispensing or expelling of the drug container even without a previous communication with the communication device.

In a further aspect the disclosure relates to a drug delivery system comprising a drug delivery device as described above. The drug delivery system further comprises a communication device. The communication device comprises a communication device interface. The communication device interface is configured or operable to receive uplink data from the delivery device interface of the drug delivery device. The communication device interface is further configured to transmit downlink data to the delivery device interface.

The communication device further comprises a communication device processor that is connected to the communication device interface. The communication device processor is operable a) to generate at least a portion of the downlink data from the uplink data and/or b) to transmit at least a portion of the uplink data to an external database via a network and to receive at least a portion of the downlink data from the external database, typically via the same network.

In this way, the communication device is either operable to generate downlink data from the uplink data and/or to transmit uplink data received form the drug delivery device to an external database and to receive downlink data from the external database in response. The processing of uplink data either by the communication device processor or by an external database enables a simplification of data processing capabilities of the drug delivery device. In one example, the drug delivery device may be limited to an identification of the drug container provided in the receptacle and to a transmission of the container identification to the communication device.

Adjusting or modifying or even generation of at least one expelling or dispensing parameter may be provided by one of the communication device processor or the external database. Computational power of the drug delivery device may be thus reduced to a minimum. This may help to reduce manufacturing and maintenance costs of the drug delivery device. Moreover, shifting data processing logic from the drug delivery device towards the communication device and/or to an external database helps to avoid executing of software updates of the drug delivery device. The lifetime of the drug delivery device may be thus extended since it may not require any software update.

If the drug delivery device is equipped with a patient identifying arrangement and when a patient identifier is incorporated in the uplink data at least one of the communication device processor and the external database is or are operable to process and to generate or to modify the downlink data in dependence of the patient identifier. In this way, the downlink data can be individualized in accordance to the patient identifier provided by the patient identifying arrangement.

With another example the communication device comprises a patient identifying arrangement connected to at least one of the communication device processor and the communication device interface. The patient identifying arrangement is operable to identify a patient using at least one of the communication device and the drug delivery device. The patient identifying arrangement of the communication device is operable to generate a patient identifier and to incorporate the patient identifier into the uplink data or to transmit the patient identifier to the communication device processor.

It is generally conceivable, that only one of the communication device and the drug delivery device is provided with a patient identifying arrangement. If the drug delivery device is provided with a patient identifying arrangement the communication device may be void of a patient identifying arrangement. If the communication device is equipped and provided with a patient identifying arrangement the drug delivery device may be void of a patient identifying arrangement. It is also conceivable, that both, the communication device and the drug delivery device are equipped with a patient identifying arrangement. Generally, the patient identifying arrangement of the communication device may be equally or similarly equipped than the patient identifying arrangement as described above in connection with the drug delivery device. All features, benefits and functions of the above described patient identifying arrangement of the drug delivery device equally apply to the patient identifying arrangement of the communication device.

The communication device is either operable to process a patient identifier locally, through the onboard communication device processor. Here, the communication device processor may be operable to generate, or to modify at least one expelling or dispensing parameter based on the patient identifier. Alternatively or additionally the communication device may be operable to transmit the patient identifier to the external database. Then, the external database is operable to process the patient identifier accordingly. Here, the external database may be operable to generate or to modify at least one expelling or dispensing parameter on the basis of the patient identifier.

According to a further example the communication device processor and the communication device interface are operable to transmit a patient retrieval request to the external database and to receive a patient specific dataset from the external database in response to the patient retrieval request. The patient retrieval request includes at least the patient identifier. In this example it is intended that the communication device itself is operable to modify at least one expelling or dispensing parameter on the basis of the patient identifier. Based on the patient identifier included in the patient retrieval request the communication device will receive a patient specific dataset from the external database. Of course, the communication between the communication device and the external database is based on an authorization. The communication device must be initially authorized to communicate with the external database and to receive data therefrom. For this, conventional authorization schemes and methods typically apply.

Transmitting of a patient retrieval request to the external database and receiving the patient specific dataset in response enables the communication device to modify the at least one expelling or dispensing parameter. This enables the communication device, in particular the communication device processor to control, to adjust, and/or to calibrate the drive mechanism. Consequently, the communication device processor may be operable to process the patient specific dataset and to derive or to modify at least one expelling or dispensing parameter of the drive mechanism from the patient specific dataset. The at least one expelling or dispensing parameter may then be incorporated into the downlink data transmitted to the drug delivery device.

The communication device may further comprise a communication device storage operable to store at least one or more of at least a portion of the container identification, at least a portion of the uplink data, at least a portion of the downlink data and/or at least a portion of the patient specific dataset. Typically, the communication device storage is operable and configured to store the entire patient specific dataset and the entirety of downlink data received from the external database or generated by the communication device processor.

The communication device, in particular the communication device storage may be operable to store numerous patient specific datasets that belong to different patients enabled and/or authorized to use the drug delivery system and/or the drug delivery device.

The drug delivery system, in particular the communication device may be operable to communicate with a plurality or with numerous drug delivery devices. Consequently, uplink data received from a first drug delivery device may also include an identifier of the respective drug delivery device. Respective downlink data may include a respective device identifier indicating, for which of the numerous drug delivery devices the downlink data is intended. In this way, one and the same communication device can be shared by a number of drug delivery devices. A number of persons, e.g. living in the same household and each of which making use of an own drug delivery device may share a common communication device to adjust, to modify or to extract at least one expelling or dispensing parameter and/or to control, to adjust or to calibrate the respective drug delivery device, e.g. on demand of a user's request.

In a further example the communication device processor and the communication device interface of the communication device are operable a) to incorporate at least a portion of the patient specific dataset into the downlink data transmitted to the delivery device interface or b) to process at least a portion of the patient specific dataset and to adjust or to modify at least one of an expelling or dispensing parameter of the drive mechanism of the drug delivery device and to incorporate the at least one expelling or dispensing parameter into the downlink data transmitted to the delivery device interface.

When patient specific data is at least partially or entirely incorporated into the downlink data the delivery device processor is typically operable to extract and to process the patient specific data from the downlink data and to control, to adjust or to calibrate the drive mechanism accordingly. Alternatively and when the communication device processor has already adjusted or modified the at least one expelling or dispensing parameter and when the respective expelling or dispensing parameter has been incorporated into the downlink data it is only required that the delivery device processor receives, extracts and deploys the expelling or dispensing parameter upon or prior to an activation of the drive mechanism. In such a situation, the functionality and the data processing capabilities of the delivery device processor can be reduced compared to a configuration, wherein patient specific data has to be processed by the delivery device processor.

In a further example the delivery device processor is operable to detect a replacement or an insertion of a drug container in or into the receptacle. The delivery device processor is further operable to trigger a transmission of a patient retrieval request to the external database via the communication device in response to such a detection of the drug container replacement or drug container insertion. The drug delivery device, the housing and/or the receptacle may be provided with a switch or sensor by way of which replacement or insertion of the drug container in or into the receptacle can be detected. The switch or sensor is connected to the delivery device processor. Upon detection of a drug container replacement or drug container insertion, a patient retrieval request is transmitted to the external database.

When inserting a new drug container into the receptacle the drive mechanism is hence automatically adjusted or calibrated. In this way, and if for instance a second drug container is inserted into the receptacle replacing a first drug container and wherein the second drug container contains a drug with a different concentration of a pharmaceutically active component compared to the first drug the patient retrieval request includes at least an identification of the drug container. The previously identified container identification is incorporated into the uplink data. Optionally, a patient identifier is retrieved from the patient identifying arrangement or a patient identifier is read or retrieved from the delivery device storage. The patient identifier may be also incorporated into the uplink data.

The uplink data is then transmitted to the communication device. The communication device may then either generate downlink data from the received uplink data or may forward the uplink data or a portion thereof to the communication device. Once the uplink data has been received by the external database the downlink data is generated or modified and is hence transmitted to the communication device and further towards the drug delivery device.

In addition or instead of triggering a transmission of a patient retrieval request to the external database the delivery device processor may be simply operable to trigger transmission of uplink data and receiving of downlink data in response to the detection of a drug container replacement or drug container insertion into the receptacle. Once a drug container replacement or drug container insertion has been detected by the drug delivery device the drive mechanism may be blocked per default until the container identification of the new drug container has been identified.

If for instance, the container identification of a second drug container replacing a first drug container is identical to the container identification of the first drug container then the container identification arrangement may be operable to release the drive mechanism right away, even without a transmission and receipt of uplink data and/or downlink data.

If the container identification of the second drug container differs from the container identification of the first drug container then, at least one of the drug container identification arrangement and the delivery device processor is operable to trigger transmission and receipt of uplink and downlink data, respectively. If container identifications of subsequently used drug containers are not identical an automated control, adjustment and/or calibration of the drive mechanism, hence adjusting or modification of at least one expelling or dispensing parameter of the drive mechanism will be triggered.

According to a further aspect the disclosure also relates to a method of adjusting at least one expelling or dispensing parameter of a drug delivery device. In particular, the method includes and provides an automated or self-acting control, adjustment and/or calibration of a drive mechanism of the drug delivery device. Here, the drug delivery device comprises a receptacle configured to accommodate a drug container. The drug container comprises a container identification. The container identification is at least indicative of a content of the drug container.

The drug delivery device further comprises a drive mechanism that is operably engageable with the drug container. The drive mechanism is configured or operable to expel or to dispense at least a dose of the drug or medicament from the drug container. The method comprises the steps of determining or detecting the container identification when the drug container is arranged in the receptacle. In particular, the method comprises the step of determining or detecting the container identification upon insertion or replacement of the drug container in or into the receptacle. The method further comprises the step of transmitting uplink data from the drug delivery device to a communication device; optionally the method comprises the step of transmitting uplink data from the communication device to an external database. The uplink data transmitted from the drug delivery device to the communication device and/or transmitted from the communication device to the external database contains at least the container identification. Optionally, the uplink data contained at least one physiologic parameter or environmental parameter. Further optionally the uplink data contained a patient identifier.

The method further comprises the step of generating or modifying downlink data at least on the basis of the uplink data and transmitting the downlink data to the drug delivery device. The downlink data or at least a portion thereof is generated or modified by at least one of a delivery device processor, a communication device processor or by the external database.

The method further comprises the step of processing the downlink data and adjusting or modifying the at least one expelling or dispensing parameter of the drive mechanism on the basis of the downlink data. Adjusting or modifying the at least one expelling or dispensing parameter may be conducted by one of the communication device processor and the delivery device processor. Alternatively, the at least one expelling or dispensing parameter of the drive mechanism is extracted from the downlink data upon processing of the downlink data. Extraction of the at least one expelling or dispensing parameter from the downlink data is either provided by the delivery device processor or by the communication device processor.

Incorporation of at least the container identification into the uplink data, transmission of the uplink data and receipt of downlink data in response enables to simplify the electronic implementation and data processing implementation of the drug delivery device.

In a further example the method comprises also the step of identifying a patient using the drug delivery device and generating a patient identifier being indicative of the patient. The patient identifier is incorporated into the uplink data. A patient specific dataset is then retrieved from an external database on the basis of the patient identifier and at least a portion of the patient specific dataset is incorporated into the downlink data. Alternatively, at least one of the expelling or dispensing parameter of the drive mechanism is adjusted or modified on the basis of the patient specific dataset. Identifying of a patient and generating a patient identifier is either provided by a patient identifying arrangement of the drug delivery device or it may be provided by a patient identifying arrangement of the communication device. Providing patient identification allows and enables a patient specific setup of the drug delivery device and to provide a patient specific control, adjustment or calibration of the drive mechanism of the drug delivery device.

In a further example the method also comprises the step of retrieving or measuring at least one physiologic or environmental parameter and to incorporate the physiologic parameter or environmental parameter into one of the uplink data or downlink data. In this way, the setting of the drug delivery device and/or the control, adjustment or calibration of the drive mechanism can take into account the at least one environmental parameter or physiologic parameter.

The above described drug delivery device and the drug delivery system as well as the communication device and the external database are particularly configured to execute the above described method of adjusting at least one expelling or dispensing parameter of the drug delivery device. Insofar all features, benefits and functions described in connection with the drug delivery device and the drug delivery system equally apply to the method of adjusting; and vice versa.

In a further aspect the present disclosure also relates to a computer program for adjusting at least one expelling or dispensing parameter of a drug delivery device. The drug delivery device comprises a receptacle to accommodate a drug container and the drug container comprises a container identification being at least indicative of a content of the drug container. The drug delivery device further comprises a drive mechanism operably engageable with the drug container and configured or operable to expel or to dispense at least a dose of the drug from the drug container.

The computer program, when deployed in a delivery device processor of the drug delivery device or when deployed in a communication device processor of the communication device, comprises computer-readable instructions configured or operable to determine or to detect the container identification when the drug container is arranged in the receptacle. The computer program further comprises computer-readable instructions configured or operable to transmit uplink data from the drug delivery device to the communication device, wherein the uplink data contains at least the container identification. The computer program further comprises computer-readable instructions configured or operable to generate downlink data at least on the basis of the uplink data and to transmit the downlink data to the drug delivery device. The computer program further comprises computer-readable instructions configured or operable to process the downlink data and to adjust or to modify the at least one expelling or dispensing parameter of the drive mechanism on the basis of the downlink data. Alternatively, the computer program comprises computer-readable instructions configured or operable to process the downlink data and to extract at least one expelling or dispensing parameter of the drive mechanism from the downlink data.

Generally, the computer program is configured or operable to execute the above described method of adjusting at least one expelling or dispensing parameter. The computer program may be implemented on both, the drug delivery device and the communication device, optionally, the computer program is implemented or implementable in the external database.

The computer program is particularly operable to deploy or to execute the above described method of adjusting at least one expelling or dispensing parameter. Insofar, all features, benefits and effects described above in connection with the drug delivery device, the communication device and with the method of adjusting at least one expelling or dispensing parameter of the drug delivery device equally apply to the computer program and vice versa.

According to another aspect there is provided a computer program configured for implementation in a communication device of a drug delivery system as described above. The computer program when deployed, implemented or executed in the communication device comprises computer-readable instructions configured or operable to receive uplink data from the drug delivery device, in particular from the delivery device interface. The computer program further comprises computer-readable instructions configured or operable to transmit downlink data to the delivery device interface. The computer program further comprises computer-readable instructions configured or operable to generate at least a portion of the downlink data from the uplink data. Additionally or alternatively the computer program comprises computer-readable instructions configured or operable to transmit at least a portion of the uplink data to an external database via a network and to receive at least a portion of the downlink data from the external database.

This computer program is particularly dedicated and configured to run exclusively on the communication device in order to set up a communication link with the drug delivery device and to process uplink data and/or downlink data.

Insofar, all features, functions and effects described above in connection with the communication device equally apply to this particular computer program.

With the above described drug delivery device, the drug delivery system and the method of adjusting at least one expelling or dispensing parameter of the drug delivery device, the communication device and hence a connected device, can be triggered to seek access to the internet and cloud-based information. The medicament provided in the drug container is automatically compared to a required medicament and the treatment scheme as prescribed by the physician. A dosing mechanism and/or a drive mechanism of the drug delivery device will adapt and the device will start delivery of the medication in the prescribed scheme. Thus the patient will be supported without further interference or need for corrective actions. Treatment information may be stored and uploaded from the connected device our communication device to the internet into the cloud-based patient profile where it can be accessed by the patient and healthcare professionals.

For example in pain therapy following surgery, there are highly potent pain medications, such as opioids used that have certain potential for addiction and misuse. Good control of dosing schemes and potential remote control of treatment by the physician may be required to keep the patient adequately treated and prevent misuse. Information of the medication and device dosing settings can be transmitted online through the communication device or connected device and adaptation of treatment can be performed in real time by remote interaction with the physician.

Such safety features can be provided by the above-described combination of design elements of the drug container, an electronic container identification, such as a NFC label, and a drug delivery device featuring a corresponding container identification arrangement, e.g. a NFC reader that initiates a control or communication mechanism in the device. In addition, connectivity of the drug delivery device and/or of a communication device to the internet enables to connect and bi-directionally exchange treatment information and updated information on therapy schedules with a personalized database.

Insofar, an automated recognition of different medications inserted into a single device, e.g. a wearable injector, that is designed for complex treatment schemes can be enabled. There are therapies where simultaneous, alternating, or subsequent administration of medication has to be performed. For example, in combination therapy of hypertension, blood pressure lowering drugs are combined with drugs blocking and stimulating the renin-angiotensin system, or with calcium antagonists or with diuretics.

In diabetes therapy, there may be a combination of insulin with its antagonist glucagon to enable good regulation of blood glucose levels in a closed loop system. In another configuration, there may be a combination of two drugs in one device - one of a rapid acting insulin for continuous basal delivery of insulin and another in a second drug reservoir to deliver a GLP1-agonist at predefined doses at certain time points during the day.

Other upcoming therapies in cancer treatment may also use multiple drugs in combination to enable advanced therapies. For example, treatment with chemotherapeutic agents can be followed by treatment with agents to stimulate the white blood cell generation.

As in the previous examples, specific medication identifiers provided on a primary drug container, such as RFID tags, NFC labels, data-matrix codes, or mechanic features, can trigger device functions or data processing. Different principles of electronic, opto-electronic or mechanic differentiators may be applied in one drug-device combination product to avoid interference. Synergies can be generated with specific body-worn sensors that measure body functions, such as blood pressure, heart rate or blood glucose levels for feedback to the device to control and regulate the drug delivery device dosing and dispensing functions according to dosing algorithms, thereby defining closed-loop systems.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a protein, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;

or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the art that various modifications and variations can be made to the present invention without departing from the scope of the invention. Further, it is to be noted, that any reference numerals used in the appended claims are not to be construed as limiting the scope of the invention.

### Brief description of the drawings

In the following, numerous examples of the drug delivery device, the drug delivery system and of a method of adjusting at least one expelling or dispensing parameter of a drug delivery device are described in detail by making reference to the drawings, in which:
- Fig. 1: shows a block diagram of one example of the drug delivery system,
- Fig. 2: shows a block diagram of another example of the drug delivery system,
- Fig. 3: is indicative of an external database and
- Fig. 4: shows a flowchart of a method of adjusting at least one expelling or dispensing parameter of the drug delivery device.

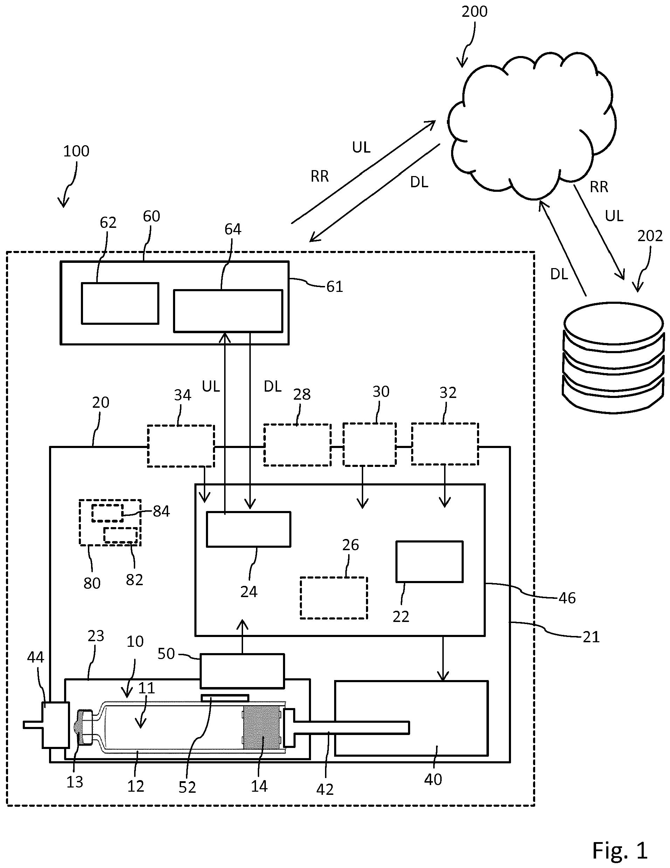

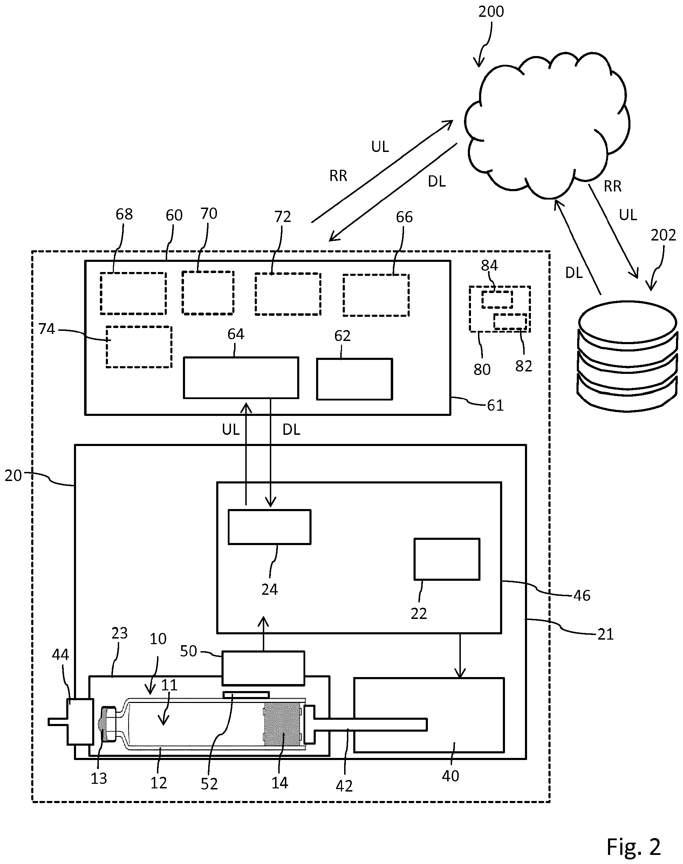

In Fig. 1, one example of a drug delivery system 100 is schematically illustrated. The drug delivery system 100 comprises a drug delivery device 20. The drug delivery device 20 comprises a housing 21. The drug delivery device 20 further comprises a receptacle 23 configured to receive and/or to accommodate a drug container 10. The drug container 10 is provided with a container identification 52. The drug delivery device 20 further comprises an outlet 44 configured for a fluid transferring engagement with the interior of the drug container. The drug container 10 contains a drug 11 or medicament. The drug 11 may be provided as a liquid drug in the interior of the drug container 10. In the illustration of Figs. 1 and 2 the drug container 10 is illustrated as a cartridge having a tubular-shaped barrel 12 extending in a longitudinal direction. The barrel 12 and hence the drug container 10 comprises a distal end provided with a seal 13. The drug container 10 comprises an opposite proximal end which is closed by a longitudinally displaceable stopper 14 or bung. The interior volume provided between the seal 13 and the stopper 14 may be entirely filled with the liquid drug. Once there is established a fluid transferring connection between the outlet 44 and the interior volume of the drug container 10 the stopper 14 can be urged in distal direction, hence towards the seal 13 in order to expel an amount, e.g. a dose of the drug 11 from the barrel 12.

In order to drive or to urge the stopper 14 in distal direction or in a dispensing direction the drug delivery device 20 comprises a drive mechanism 40. The drive mechanism 40 comprises a driver 42, which may be implemented as a plunger or piston rod configured to exert a distally directed pressure onto the stopper 14 in order to drive the same in distal direction during dose expelling or dose dispensing.

The presently illustrated drive mechanism 40 and the drug container 10 are only exemplary for a large variety of drug containers and dispensing or drive mechanisms by way of which a drug can be expelled or dispensed from a drug delivery device. With other examples the drive mechanism 40 may include a pump, such as a peristaltic pump. Alternatively, the drive 40 mechanism may be implemented as a spray mechanism configured to expel or dispense the drug by way of spraying. The drug container 10 may comprise a flexible bag or a flexible pouch filled with a liquid medicament or filled with a lyophilized drug. A flexible or deformable drug container is particularly configured for coupling with a drive mechanism 40 implemented as a pump. Here, the liquid medicament can be withdrawn from the drug container 10 by a suction force.

The drug container 10 may also comprise a drug or medicament in powdered form configured for producing an aerosol or configured for spraying.

Depending on the combination of drug container 10 and a correspondence drive mechanism 40 the drug delivery device 20 may be configured or provided as an injection device, e.g. as a pen-type injector, as an injection pump or infusion pump or as an inhaler.

The outlet 44 may thus represent an injection needle or a connector for a fluid transferring connection to an injection cannula or an infusion tube. Alternatively, the outlet 44 may represent a mouthpiece, e.g. when the drug delivery device 20 is implemented as an inhaler.

The drug delivery device 20 comprises a container identification arrangement 50 that is configured to detect and/or to identify the container identification 52 provided on the drug container 10 when the drug container 10 is arranged inside the receptacle 23 of the drug delivery device. The container identification 52 comprises one of a mechanical, optical, capacitive, magnetic and/or electromagnetic encoding that can be detected and read by the container identification arrangement 50. The container identification arrangement 50 is further configured to communicate at least with a delivery device interface 24 of the drug delivery device 20. In this way, a container identification 52 retrieved or detected by the container identification arrangement 50 can be transmitted or transferred to the delivery device interface 24.

The delivery device interface 24 is configured to transmit uplink data UL to a communication device 60. The uplink data UL contains at least the container identification 52 that has been obtained from the container identification arrangement 50. The delivery device interface 24 is further configured to receive downlink data DL from the communication device 60 in return for the transmission of the uplink data UL to the communication device 60.

The drive mechanism 40 which is operably engageable with the drug container 10 is e.g. configured to expel or to dispense at least a dose of the drug 11 from the drug container 10 on the basis of the downlink data DL received from the delivery device interface 24. Insofar, the delivery device interface is configured to control, to adjust or to calibrate the drive mechanism 40 upon receipt of downlink data DL.

Typically, the drug delivery device 20 comprises a delivery device processor 22 that is connected to the delivery device interface 24 and which is further operable to control the drive mechanism 40 on the basis of the downlink data DL received from the communication device 60 and hence from the delivery device interface 24.

In particular, the delivery device processor 22 is operable to process downlink data DL that has been obtained from the delivery device interface 24 and to adjust or to modify at least one expelling or dispensing parameter of the drive mechanism 40 on the basis of the downlink data DL. Alternatively, the delivery device processor 22 is operable to extract at least one expelling or dispensing parameter of the drive mechanism 40 from the downlink data DL.

There are several options of the data structure of the downlink data. One option is that the downlink data DL contains drug container specific information that has to be translated into or mapped to an expelling or dispensing parameter that can be processed by the drive mechanism. For this, the delivery device processor 22 is configured to adjust or to modify the at least one expelling or dispensing parameter of the drive mechanism on the basis of the downlink data DL.

In other examples the expelling or dispensing parameter which may be directly processible by the drive mechanism 40 is already contained in the downlink data DL received from the communication device 60 and/or from the delivery device interface 24. In this case, the delivery device processor 22 only has to extract the at least one expelling or dispensing parameter from the downlink data DL and to forward the expelling or dispensing parameter to the drive mechanism 40 typically during dispensing or expelling of a dose of the drug from the drug container 10.

The at least one expelling or dispensing parameter provided to the drive mechanism may define a travelling distance or travelling duration of the driver 42 of the drive mechanism 40. The at least one expelling or dispensing parameter may be further indicative of a point of time at which an expelling or dispensing action has to start and/or a point of time at which a expelling or dispensing action should terminate. The expelling or dispensing parameter may be further indicative of at least one of a driving velocity and a driving force of a driver 42 of the drive mechanism 40. Typically, the at least one expelling or dispensing parameter is directly indicative of an operating parameter of the drive mechanism.

Uplink data UL may be limited to a container identification. Depending on the container identification, e.g. provided to an external database 202 or provided to a communication device 60 the downlink data DL is generated. The downlink data DL may include further information of the identified drug container 10. The downlink data DL may include drug container specific supplemental information, such as information about the drug or medicament, information about the concentration of a pharmaceutically active component in the drug or medicament, information about the durability of the drug, information about the production date. The downlink data DL may further contain information about a treatment schedule according to which the drug or medicament should be administered to a patient. The downlink data DL may be indicative of a treatment schedule and may contain information about a point of time at which a certain amount of the medicament or drug or of the pharmaceutically active component should be dispensed or expelled by the drive mechanism.

With one example the uplink data UL only contains a container identification. The communication device 60 may be configured to generate respective downlink data DL that is compliant with a prescribed treatment schedule. Here, a treatment schedule may be provided in the communication device 60 or by an external database 202. If for instance a treatment schedule is provided by an external database 202 the communication device 60 is particularly operable to transmit at least a portion of the uplink data UL via a network 200 to the external database 202. In response, the external database 202 is configured to transmit downlink data DL on the basis of received uplink data via the network 200 back to the communication device 60.

With some examples the entire uplink data UL as provided by the container identification arrangement 50 is unalterably transmitted to the external database 202 or only a portion of the uplink data UL as provided by the delivery device interface 24 is routed or transmitted to the database 202 whereas another portion of the uplink data UL is locally processed by the communication device 60. Accordingly, the entire downlink data or at least a portion thereof may be provided by the database 202 and may be transmitted to the communication device 60. Another portion of the downlink data to be transmitted from the communication device 60 to the drug delivery device 20 may be locally generated or processed by the communication device 60.

The downlink data received from the database 202 at the communication device 60 may include a delivery schedule, e.g. specifying only the day and time of a drug administration and an amount of the pharmaceutically active substance that should be administered to the patient at the given day and time.

From this information obtained from the database 202 at the communication device 60 the communication device 60, in particular the communication device processor 62 may be operable to derive at least one expelling or dispensing parameter specific to the drug delivery device 20. The container identification 52 may be further indicative of a concentration of the pharmaceutically active compound in the drug as provided in the drug container 10. Depending on this concentration information, the communication device 60, in particular its processor 62 may be operable to modify the at least one expelling or dispensing parameter on the basis of the drug concentration information and on the basis of the total amount of the pharmaceutically active compound that should be administered.

In one example and when a first drug container 10 is used containing a first drug with a first concentration of a pharmaceutically active compound the at least one expelling or dispensing parameter of the drive mechanism defines, that for a delivery of a prescribed amount of the pharmaceutically active compound the driver 42 of the drive mechanism 40 has to be moved by a first distance.

Now and on the basis of a second container identification 52 of a second drug container 10 filled with a second medicament having a different, e.g. a lower concentration of the pharmaceutically active compound the at least one expelling or dispensing parameter of the drive mechanism will be adjusted or modified accordingly. In order to administer the required amount of the pharmaceutically active compound, the displacement of the driver 42 has to be modified, e.g. increased. Calculating of such modifications of expelling or dispensing parameters is provided by one of the delivery device processor 22 and the communication device processor 62. There are also examples, wherein the adjusting or modification of such expelling or dispensing parameters is conducted and provided by the external database 202.

In situations, wherein the expelling or dispensing parameter for the drive mechanism 40 is adjusted or modified on the basis of the uplink data UL by the communication device processor 62 or by the external database 202 the delivery device processor 22 only has to extract the at least one expelling or dispensing parameter from the downlink data DL received from the communication device 60.

If the adjustment or modification of the at least one expelling or dispensing parameter of the drive mechanism 40 is provided by the external database 202 the functionality of the communication device 60 can be reduced to a mere transmission of uplink data and downlink data between the external database 202 and the drug delivery device 20. With some examples, the communication device 60 is operable to receive uplink data UL from the drug delivery device 20 and to forward the uplink data UL unalterably to the external database 202. With the same or with further examples the communication device 60 may be operable to receive downlink data DL from the external database 202 and to forward the downlink data DL unalterably to the drug delivery device 20.

With other examples the communication device is operable to derive or to generate at least a portion or the entire downlink data from the uplink data received from the drug delivery device. With other examples, the communication device 60 is operable and configured to process a portion of the uplink data UL received from the drug delivery device 20 and to forward another portion of the uplink data UL to the external database. The communication device 60 is then further operable to receive downlink data DL from the external database 202 and to combine or to incorporate the received downlink data from the external database 202 with downlink data DL generated by the communication device 60.

The presently proposed architecture of a drug delivery system comprising a drug delivery device capable of identifying a container identification of a drug container and further operable to generate and to transmit uplink data to a communication device provides numerous possibilities of data processing for the purpose of providing a rather automated control, adjustment or calibration of the drive mechanism 40 of the drug delivery device.

With typical examples the drug delivery device 20 comprises an electronic circuit 46. The electronic circuit 46 may be implemented as an integrated electronic circuit. The electronic circuit 46 may comprise the above described delivery device processor 22 and the delivery device interface 24. The delivery device interface 24 and the delivery device processor 22 may be implemented on one and the same chip or on one and the same integrated circuit.

Optionally, the electronic circuit 46 and hence the drug delivery device comprises a delivery device storage 26. The delivery device storage 26 may be implemented as a non-volatile memory. The delivery device storage 26 is typically configured or operable to store at least one or more of at least a portion or the entirety of the container identification 52, at least a portion or the entirety of uplink data UL transmitted from the delivery device interface 24 to the communication device 60 and at least a portion or the entirety of downlink data DL received from the communication device 60. In this way, downlink data as well as the at least one expelling or dispensing parameter can be locally stored in the drug delivery device 20. This ensures a reliable and failure safe operation of the drug delivery device 20 even if a communication link with the communication device 60 should not be available.

The communication device 60 comprises at least a communication device processor 62 and a communication device interface 64. The communication device interface 64 is configured and operable to set up a communication link with the delivery device interface 24. Typically, the communication device interface 64 and the delivery device interface 24 are configured and operable to establish or to set up a wireless communication link operable to transmit uplink data UL and downlink data DL between the drug delivery device 20 and the communication device 60. The wireless communication between the communication device 60 and the drug delivery device may be based on standardized RF communication protocols, such as Bluetooth, NFC or Wi-Fi standards or any other standardized communication protocol enabling a wireless electromagnetic data transmission between the drug delivery device 20 and the communication device 60.

Alternatively, there may be provided a wired communication link between the communication device 60 and the drug delivery device 20.

The drug delivery device as illustrated in Fig. 1 comprises numerous optional components indicated with dashed rectangles. As illustrated in Fig. 1, the delivery device storage 26 is provided only optionally. The drug delivery device 20 may further comprise a sensor assembly 80 configured to measure at least one physiologic parameter or environmental parameter and being further configured to transmit the at least one physiologic parameter or environmental parameter to the delivery device processor 22. The sensor assembly 80 comprises at least one sensor 82 and a sensor interface 84. The sensor interface 84 is operable to transmit the at least one physiologic or environmental parameter to the delivery device processor 22 or to the delivery device interface 24.

The sensor assembly 80 may be integrated into the drug delivery device 20. The drug delivery device 20 and/or its housing 21 may be configured and suitable to be worn on the body or skin of a patient as it is the case for, e.g. an infusion pump. The sensor assembly 80 may also be provided as a separate component of the drug delivery device located outside the housing 21. It may be connected to the drug delivery device 20 either by a wired connection or wirelessly in order to provide the at least one physiologic or environmental parameter to the drug delivery device 20. Here, the at least one physiologic or environmental parameter can be used to further adjust or to modify the at least one expelling or dispensing parameter.

The sensor assembly 80 can be for instance implemented as a blood glucose measuring sensor providing regular electronic signals being indicative of a blood glucose level of the patient. Depending on the respective physiologic parameter the delivery device processor 22 may be operable to adjust or to modify the amount of medicament or a delivery rate at which the drug or medicament is expelled or dispensed. The delivery device processor 22 and the sensor assembly 80 may be configured in a closed loop configuration. Hence, the processor 22 and hence the drive mechanism 40 controlled by the delivery device processor 22 may be operated or driven at least adjusted or calibrated in response to a regular measurement of the sensor assembly 80.

Optionally, the drug delivery device 20 further comprises a patient identifying arrangement 34. The patient identifying arrangement 34 is connected to at least one of the delivery device processor 22 and the delivery device interface 24. As described above, the patient identifying arrangement 34 is operable to identify a patient using the drug delivery device 20. The patient identifying arrangement is further operable to generate a patient identifier and to incorporate the patient identifier into the uplink data. The incorporation of the patient identifier into the uplink data UL may be further conducted by one of the delivery device interface 24 and the processor 22 upon receipt of the patient identifier.

Providing the uplink data UL with a patient identifier enables to receive patient specific downlink data DL from the communication device 60 and/or from the external database 202.

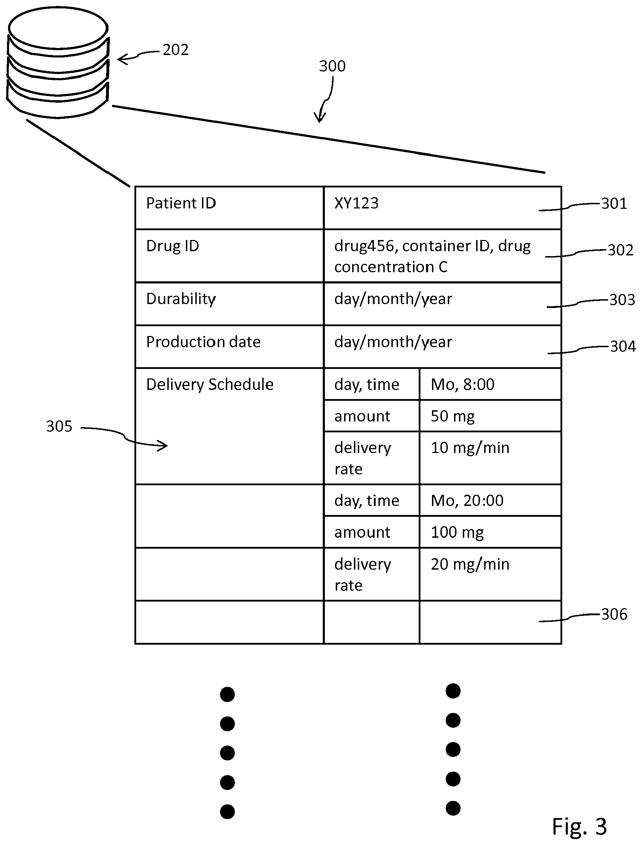

The downlink data DL may therefore include a patient specific dataset 300 as illustrated in Fig. 3, which patient specific dataset is typically stored in the external database 202 that is provided to the communication device 60 upon a patient retrieval request RR. The patient specific dataset 300 is typically incorporated into the downlink data DL generated and provided by the external database 202.

Insofar, the patient identifier enables a patient specific control, adjustment and calibration of the drive mechanism as well as a patient specific use of the drug delivery device 20 or of the drug delivery system 100. This principally enables to offer use of one and the same drug delivery device 20 and/or drug delivery system 100 to a group of users. These users may share at least one of a drug delivery device 20, a communication device 60 and a drug delivery system 100. If the patient identifying arrangement 34 recognizes or asserts that a first user is actually using the drug delivery device 20 a respective patient retrieval request is transmitted to the external database 202. Then, the patient specific dataset 300 is incorporated into the downlink data DL and the operation of the drug delivery device 20, e.g. the control, adjustment or calibration of the drive mechanism 40 is adjusted in accordance to the patient specific dataset.

The delivery device storage 26 of the drug delivery device 20 is of course operable to store the patient specific dataset 300 and/or to store the patient specific expelling or dispensing parameters derived from the downlink data DL that includes the patient specific dataset 300. If the patient identifying arrangement 34 identifies a different person or patient, hence a second patient intending to use the drug delivery device 20 a further patient retrieval request is transmitted to the external database 202. Accordingly, the respective patient specific dataset for the second patient will be transmitted to the drug delivery device 20 and the patient specific settings of the drug delivery device; in particular a control, adjustment or calibration of the drive mechanism 40 will be adapted accordingly.

Also here, the patient specific dataset and/or patient specific expelling or dispensing parameters may be locally stored in the delivery device storage 26. If the drug delivery device 20 is then repeatedly used by the first patient, the delivery device processor 22 may be operable to resume the previously stored patient specific data or patient specific expelling or dispensing parameters of the first patient from the delivery device storage 26 without conducting a further patient retrieval request RR.

The drug delivery device 20 may further comprise an indicator 28 or a display informing the user of drug delivery specific parameters or information. The indicator 28 or display may visually, audibly or haptically indicate to the user or patient that a certain size of a dose is actually set and is due for dispensing or expelling within a certain timeframe. The indicator 28 or display may further inform or alert the user about a malfunction of the drug delivery device 20. The indicator 28 may further remind the user of undertaking some delivery related actions, such as setting of a dose by using an optional dose dial 30 and/or to trigger dispensing or expelling of a dose of the drug by using an optional trigger 32.

With the example as illustrated in Fig. 1 numerous implementations of the drug delivery systems 100 are conceivable. Here, the functionality of the communication device 60 may be reduced to only provide a communication link between the external database 202 and the drug delivery device 20. The computational logic and computational power for adjusting or modifying the at least one expelling or dispensing parameter may be entirely provided by the drug delivery device 20 itself or by the external database 202.

With some examples the database 202 is not an external database but belongs to the drug delivery system 100.

After the drug delivery device has received downlink data DL from the database 202 and after the drive mechanism 40 has been adjusted or calibrated accordingly the drug delivery device 20 may operate in a stand-alone mode without any further interaction with the communication device 60 and/or with the external database 202.

In Fig. 2 another example of the drug delivery system 100 is illustrated. There, the drug delivery device 20 may be void of all optional components 26, 34, 28, 30, 32 and 80 as described in connection with Fig. 1. Instead, the communication device 60 may be provided with a patient identifying arrangement 74 providing the same or similar functionality as the patient identifying arrangement 34 of the drug delivery device as described above. The communication device 60 may be optionally provided with a communication device storage 66 providing all or similar functionalities compared to the delivery device storage 26 as described before in connection with Fig. 1. The communication device 60 may be further provided with at least one of an indicator 68, a dose dial 70 and a trigger 72 providing the same or similar functionalities as the indicator 28, the dose dial 30 and the triggers 32 as described in connection with Fig. 1. Additionally, the sensor assembly 80 may be provided as a separate component, which is separate from both, the drug delivery device 20 and the communication device 60. With other but non-illustrated examples the sensor assembly 80 may be integrated into the communication device 80.

With the example of Fig. 2, processing of uplink data received by the communication device interface 64 may be entirely or at least partially conducted by the communication device processor 62. Uplink data received from the delivery device interface 24 may be further provided with a patient identifier obtained from the patient identifying arrangement 74 and modified uplink data UL will be transmitted from the communication device 60 via the network 200 to the external database 202.

With typical examples the communication device 60 is implemented as a smartphone, as smartwatch or as a tablet computer. The communication device 60 is typically a mobile electronic device. It may be provided with a software application or with a software configured to establish communication with the external database 202 and further configured to establish communication with the drug delivery device 20. With some examples, the drug delivery device 20 may be void of any manually operable actuation members, such as buttons or dials. Instead, the setting of a dose as well as dispensing of a dose may be entirely controlled and triggered via the communication link between the communication device 60 and the drug delivery device 20. For this, the communication device 60 may provide an emulated dose dial 70 and/or an emulated trigger 72.

It should be noted, that the examples of Fig. 1 and Fig. 2 represent two extreme implementations of the drug delivery system 100. The present disclosure also covers any further implementations of the drug delivery system 100, wherein, when starting from the implementation of Fig. 1 only one of the optional components 26, 28, 30, 32, 34, 80 is provided or implemented externally or in the communication device 60 in form of a corresponding optional component 66, 68, 70, 72, 74.

In Fig. 3 a patient specific dataset 300 as it is stored in and provided by the external database 202 is schematically illustrated. The patient specific dataset 300 comprises numerous data fields 301, e.g. representing a patient identifier or a data field 302, e.g. representing a container identification. The container identification 302 may be indicative of a drug name, a drug concentration as well as of a unique LOT or batch number. The patient specific dataset 300 may further comprise a data field 303 defining a particular date representing a best before date or a durability date until which the drug or medicament can be used. There may be further provided a data field 304 indicating the production date of the drug or medicament.

The patient specific dataset 300 may further comprise a data field 305 representing and/or providing a delivery schedule. The delivery schedule 305 may represent numerous dates and/or times at which a certain amount of the medicament should be administered at a given or prescribed delivery rate.

Medical staff or caregivers typically have access to the external database 202. In this way, a medicament prescription schedule and hence a delivery schedule can be stored in the external database 202. The external database 202 is typically accessible via a network 200. Insofar the database 202 may be physically located in or represented by a data cloud.

Moreover, the patient specific dataset 300 may comprise another data field 306 for storing feedback data obtained from the drug delivery device, e.g. about a successful completion of a drug administering procedure, a confirmation message may be automatically generated and transmitted from one of the drug delivery device 20 and the communication device 60 to the external database 202. Insofar, treatment data can be automatically uploaded to the external database and to a data cloud.

With the presently proposed drug delivery system and the associated infrastructure it is generally conceivable to implement a large variety of different scenarios of use.

For instance, one of the drug delivery device 20 and the communication device 60 may be preprogrammed to trigger transmission of a patient retrieval request RR at regular time intervals, e.g. once a week. This offers the possibility for a healthcare provider to modify the patient specific dataset if required. In response to the patient retrieval request or when a drug container is replaced in the drug delivery device 20 a new calibration and adjustment of the drive mechanism 40 is automatically conducted on the basis of the downlink data DL received in response to the patient retrieval request RR or in response to the transmission of uplink data UL that is automatically triggered in response to the replacement of a drug container. In this way, the delivery schedule can be dynamically changed, e.g. via a modification of patient specific data in the database 202. In order to change a treatment schedule no hardware or software modifications have to be made with the drug delivery device or with the communication device 60.

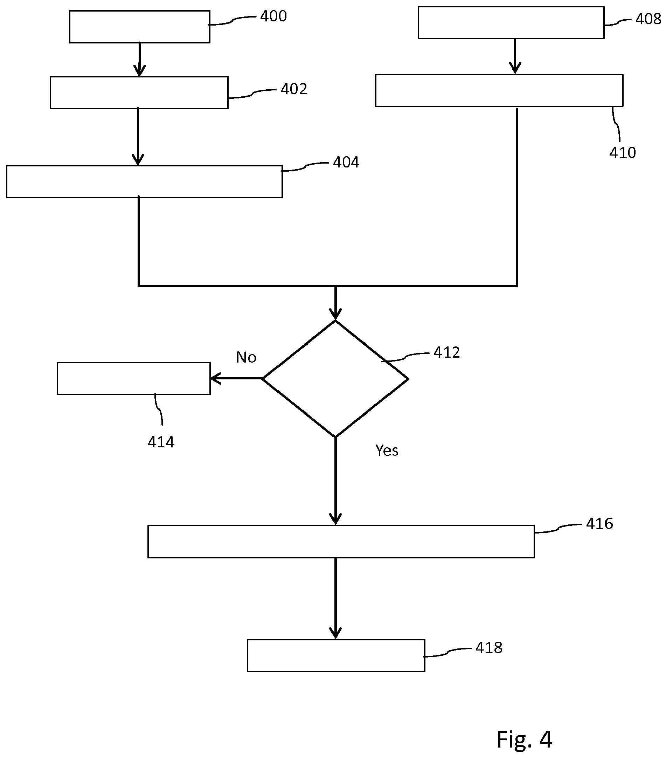

In Fig. 4, only one of a plurality of examples of a flowchart according to the presently described method of adjusting at least one expelling or dispensing parameter of a drug delivery device is illustrated. In a first step 400 a patient ID is gathered, e.g. by the patient identifying arrangement 34, 74 as provided by the drug delivery device 20 according to Fig. 1 or as provided by the communication device 60 as illustrated in Fig. 2. Thereafter, and based on the patient identifier in step 402 a patient specific dataset is requested from the external database 202. Hence, a delivery schedule for a patient with the respective patient ID is requested. In a subsequent step 404 the delivery schedule is derived from the patient specific dataset as provided to the communication device 60 and/or to the drug delivery device 20. Concurrently with steps 400, 402 and 404 the drug container 10 as located inside the receptacle 23 is identified in step 408. The identification is conducted by the container identification arrangement 50. In step 410, the identified or derived container identification is provided to at least one of the delivery device processor 22 and the communication device processor 62.

In a subsequent step 412 the delivery schedule as provided in step 404 and the container identification as provided in step 410 are mutually compared. In step 412 it is checked whether the container ID and the respective drug provided by the container matches with or is suitable for the delivery schedule as provided in step 404. If it is determined in step 412, that the drug 11 as identified in step 410 does not match with the prescribed delivery schedule as provided in step 404, the method continues with step 414. There, an alert, e.g. a visual alert, an audible alert and/or a haptic alert is generated and/or delivery of the drug by the drive mechanism 40 is blocked. For instance, the drive mechanism 40 may be locked in step 414. The alert is an indication to the user to change the drug container.

Thereafter, at least steps 408 and 410 have to be processed again. If in step 412 it then turns out, that the identified drug container and the respective drug matches with the delivery schedule as provided in step 404 then the method continues with step 416. Here, at least one expelling or dispensing parameter of the drive mechanism 40 is adjusted or modified on the basis of the data obtained in at least one of the steps 404, 410. In step 416 the drive mechanism may be adjusted or calibrated in view of, e.g. the drug concentration provided in the drug container. Thereafter and in an optional step the prescribed drug or medicament is expelled or dispensed actively or drug expelling or drug dispensing is no longer blocked.

### Reference numbers

- 10: drug container
- 11: drug
- 12: barrel
- 13: seal
- 14: stopper
- 20: drug delivery device
- 21: housing
- 22: delivery device processor
- 23: receptacle
- 24: delivery device interface
- 26: delivery device storage
- 28: indicator
- 30: dose dial
- 32: trigger
- 34: patient identifying arrangement
- 40: drive mechanism
- 42: driver
- 44: outlet
- 46: electronic unit
- 50: container identification device
- 52: container identification
- 60: communication device
- 61: housing
- 62: communication device processor
- 64: communication device interface
- 66: communication device storage
- 68: indicator
- 70: dose dial
- 72: trigger
- 74: patient identifying arrangement
- 80: sensor assembly
- 82: sensor
- 84: interface
- 100: drug delivery system
- 200: network
- 202: database
- 300: data set

## Claims

1. A drug delivery device (20) comprising:
- a receptacle (23) to accommodate a drug container (10), wherein the drug container (10) is provided with a container identification (52), the container identification (52) being at least indicative of a content of the drug container (10),
- a container identification arrangement (50) configured to detect and/or to identify the container identification (52) when the drug container (10) is arranged in the receptacle (23),
- a delivery device interface (24) configured to transmit uplink data (UL) to a communication device (60), wherein the uplink data (UL) contains at least the container identification (52) obtainable from the container identification arrangement (50) and wherein the delivery device interface (24) is configured to receive downlink data (DL) from the communication device (60) in return to the transmission of the uplink data (UL),
- a drive mechanism (40) operably engageable with the drug container (10) and configured to expel or to dispense at least a dose of the drug from the drug container (10) on the basis of the downlink data (DL) received from the delivery device interface (24).

2. The drug delivery device according to claim 1, further comprising a delivery device processor (22) connected to the delivery device interface (24) and operable to control the drive mechanism (40) on the basis of at least one expelling or dispensing parameter, wherein the processor (22) is operable:
a) to process downlink data (DL) obtained from the delivery device interface (24) and to adjust or to modify the at least one expelling or dispensing parameter of the drive mechanism (40) on the basis of the downlink data (DL), or
b) to extract the at least one expelling or dispensing parameter of the drive mechanism (40) from the downlink data (DL).

3. The drug delivery device according to any one of the preceding claims, further comprising a delivery device storage (26) configured to store at least one or more of: at least a portion of the container identification (52), at least a portion of the uplink data (UL) and at least a portion of the downlink data (DL).

4. The drug delivery device according to any one of the preceding claims, further comprising a patient identifying arrangement (34) connected to at least one of the delivery device processor (22) and the delivery device interface (24), the patient identifying arrangement (34) being operable to identify a patient using the drug delivery device (20), to generate a patient identifier and to incorporate the patient identifier into the uplink data (UL).

5. The drug delivery device according to any one of the preceding claims 2 to 4, wherein the delivery device processor (22) is further configured to process at least one physiologic parameter or environmental parameter and wherein the delivery device processor (22) is configured to adjust or to modify the at least one expelling or dispensing parameter of the drive mechanism (40) on the basis of at least one of the physiologic parameter or environmental parameter.

6. The drug delivery device according to claim 5, further comprising a sensor assembly (80) configured to measure the at least one physiologic parameter or environmental parameters and to transmit the at least one physiologic parameter or environmental parameter to the delivery device processor (22).

7. The drug delivery device according to any one of the preceding claims, wherein the container identification arrangement (50) comprises at least one of a mechanical sensor, an optical sensor, a capacitive sensor, a magnetic sensor and an electromagnetic sensor and wherein the container identification arrangement (50) is operable to generate and to transmit an electronic identification signal being indicative of the container identification (52) when the drug container (50) is arranged in the receptacle (23).

8. The drug delivery device according to any one of the preceding claims further comprising a drug container (10) arranged in the receptacle (23) and filled with drug or medicament.

9. A drug delivery system (100) comprising a drug delivery device (20) according to any one of the preceding claims and further comprising a communication device (60), the communication device (60) comprising:
- a communication device interface (64) configured to receive uplink data (UL) from the delivery device interface (24) and configured to transmit downlink data (DL) to the delivery device interface (24),
- a communication device processor (62) connected to the communication device interface (64), the communication device processor (62) being operable:
a) to generate at least a portion of the downlink data (DL) from the uplink data (UL), and/or
b) to transmit at least a portion of the uplink data (UL) to an external database (202) via a network (200) and to receive at least a portion of the downlink data (DL) from the external database (202).

10. The drug delivery system (100) according to claim 9, wherein the communication device comprises a patient identifying arrangement (74) connected to at least one of the communication device processor (62) and the communication device interface (64), the patient identifying arrangement (74) being operable to identify a patient using at least one of the communication device (60) and the drug delivery device (20), the patient identifying arrangement (74) being operable to generate a patient identifier and to incorporate the patient identifier into the uplink data (UL) or to transmit the patient identifier to the communication device processor (62).

11. The drug delivery system (100) according to claim 9 or 10, wherein the communication device processor (62) and the communication device interface (64) are operable to transmit a patient retrieval request (RR) to the external database (202) and to receive a patient specific data set (300) from the external database (202) in response to the patient retrieval request (RR), wherein the patient retrieval request (RR) includes at least the patient identifier.

12. The drug delivery system (100) according to claim 11, wherein the communication device processor (62) and the communication device interface (64) are operable:
a) to incorporate at least a portion of the patient specific data set (300) into the downlink data (DL) transmitted to the delivery device interface (24), or
b) to process at least a portion of the patient specific data set (300) and to adjust or to modify at least one of an expelling or dispensing parameter of the drive mechanism (40) of the drug delivery device (20) and to incorporate the at least one expelling or dispensing parameter into the downlink data (DL) transmitted to the delivery device interface (24).

13. The drug delivery system (100) according to any one of the preceding claims 9 to 12, wherein the delivery device processor (22) is operable to detect a replacement or an insertion of a drug container (10) in or into the receptacle (23) and wherein the delivery device processor (22) is further operable to trigger a transmission of a patient retrieval request (RR) to the external database (202) via the communication device (60) in response to a detection of the drug container replacement or drug container insertion.

14. A method of adjusting at least one expelling or dispensing parameter of a drug delivery device, the drug delivery device (20) comprising a receptacle (23) to accommodate a drug container (10), the drug container (10) comprising a container identification (52) being at least indicative of a content of the drug container (10), the drug delivery device further comprising a drive mechanism (40) operably engageable with the drug container (10) and configured to expel or to dispense at least a dose from the drug container, the method comprises the steps of:
- determining or detecting the container identification (52) when the drug container (10) is arranged in the receptacle (23),
- transmitting uplink data (UL) from the drug delivery device (20) to a communication device (60), wherein the uplink data (UL) contains at least the container identification (52),
- generating downlink data (DL) at least on the basis of the uplink data (UL) and transmitting the downlink data (DL) to the drug delivery device (20),
- processing the downlink data (DL) and adjusting or modifying the at least one expelling or dispensing parameter of the drive mechanism (40) on the basis of the downlink data (DL), or
- processing the downlink data (DL) and extracting the at least one expelling or dispensing parameter of the drive mechanism (40) from the downlink data (DL).

15. The method of claim 14 further comprising the step of:
- identifying a patient using the drug delivery device (20) and generating a patient identifier being indicative of the patient,
- incorporating the patient identifier into the uplink data (UL),
- retrieving a patient specific data set (300) from an external database (202) on the basis of the patient identifier,
- incorporating at least a portion of the patient specific data set (300) into the downlink data (DL), or
- adjusting or modifying at least one of an expelling or dispensing parameter of the drive mechanism (40) of the drug delivery device (20) on the basis of the patient specific data set (300).
